# EUROPEAN PATENT APPLICATION

(11) **EP 1 219 362 A1**
(43) Date of publication of application: **03.07.2002**
(21) Application number: 00940883.2
(22) Date of filing: 29.06.2000
(51) Int. Cl.: B09B 3/00, B09B 5/00, C02F 11/02, C02F 3/06

(54) **APPARATUS FOR TREATING GARBAGE**

(30) Priority: 29.06.1999 JP 18413799; 29.09.1999 JP 27710299
(71) Applicant: Sanyo Electric Co., Ltd., Moriguchi-shi, Osaka 570-8677 (JP)
(72) Inventor: YAMADA, Atsushi Sanyo Electric Co., Ltd., Osaka 570-8677 (JP); SUZUKI, Haruhiko Sanyo Electric Co., Ltd., Osaka 570-8677 (JP); SEKIGUCHI, Tatsuhiko Sanyo Electric Co., Ltd., Osaka 570-8677 (JP)
(74) Representative: Cross, Rupert Edward Blount
(86) International application number: JP0004303
(87) International publication number: WO0100342

(57) **Abstract**

An apparatus for treating garbage, characterized as having a flow rate controlling tank for storing temporarily a mixture of a garbage having been pulverized by a disposer with a kitchen waste, water, a solid-liquid separating device for separating the mixture supplied form the flow rate controlling vessel into a solid part and a liquid part, a composting facility for converting the solid part separated by the solid-liquid separating device into a compost, a precipitation-separation tank for precipitating fine particles in the liquid part supplied from the solid-liquid separating device, a split-flow device for distributing a liquid part supplied from the precipitation-separation tank, and a waste water treating device for subjecting a liquid part supplied form the split-flow device to a biological treatment to thereby obtain a treated water. The apparatus for treating garbage can be used for treating a mixture from a disposer with good efficiency.

## Description

### BACKGROUND OF THE INVENTION

### Field of the Invention

The present invention relates to a waste treatment apparatus comprising a solid-liquid separating device for separating a mixture composed of a solid substance and a liquid substance, the solid-liquid separating device subjecting the mixture to filtering to discharge organic waste water while decomposing organic solid substances by the action of microorganisms existing in the organic solid substance.

### Description of Related Art

Methods of treating organic waste water using aerobic microorganisms have been actively studied and developed. For example, an activated sludge method is usually used in sewage treatment plants, and a submerged infiltration bed is used along with the activated sludge method in combined waste water purifying tanks.

In addition, methods in which domestic kitchen garbage or the like is fragmented or liquefied by a disposal unit so that it can be treated as waste water containing garbage have been also developed.

For example, Japanese Patent Laid-Open Publication No. Hei 9-1117 discloses an apparatus in which waste water containing garbage is introduced into a solid substance treating section which decomposes a solid substance by means of microorganisms and discharges primary treated water, which is then introduced into a waste water treating tank for aeration. In this manner, solid substances are decomposed and removed from the waste water containing garbage from the garbage disposal, and the waste water is purified.

In the apparatus disclosed in the above publication, waste water is subjected to aeration by an air diffuser in the waste water treating tank, which is a treatment basically similar to the activated sludge method.

The present inventors, on the other hand, found that more effective purification of waste water can be performed when a waste water treating device (a secondary treating device) comprising a packed layer which is packed with microorganism carriers is provided downstream of the solid substance treating device (a primary treating device). (See Japanese Patent Laid-Open Publication No. Hei 11-19674)

In a known waste water treating apparatus , air is circulated using a gas supply member, such that the optimal oxygen concentration for the aerobic treatment is maintained in the microorganism carriers. However, in order to circulate air, in addition to the gas supply member, an air pump and a support layer or the like into which air is forced by the gas supply member must be provided, and this disadvantageously increases the apparatus in size.

Further, inclusion of an air pump increases cost and problems of noise and vibration are caused by the operation of the air pump.

In addition, because a foam glass is used as a support layer, composting and recycling is difficult, which is not preferable from environmental standpoint.

### SUMMARY OF THE INVENTION

The present invention was conceived in view of the aforementioned problems of the related art and aims to provide an apparatus for treating garbage, or a waste treatment apparatus, capable of effectively treating organic waste water such as the output of a garbage disposal or other kitchen waste water, by means of an aerobic treatment using natural diffusion of oxygen.

In accordance with one aspect of the present invention, a waste treatment apparatus comprises a disposal for mechanically reducing waste discharged from a kitchen; a flow rate controlling tank for temporarily storing a mixture of the waste reduced by said disposal and kitchen waste water; a solid-liquid separating device for separating the mixture supplied from the flow rate controlling tank into a solid component and a liquid component; a composting device for converting the solid component separated by the solid-liquid separating device into a compost; a precipitation-separation tank for precipitating fine particles in the liquid component supplied from the solid-liquid separating device; a split-flow device for distributing the liquid component supplied from the precipitation-separation tank; and a waste water treating device for biologically treating the liquid part supplied from the split-flow device to thereby produce treated water.

Said waste water treating device may comprise a tank containing microorganism carriers, and said liquid component may be introduced into the tank wherein it is subjected to a biological treatment, and from which it is then discharged out of said garbage treating apparatus.

Said waste water treating device may have perforated containers which contain said microorganism carriers.

Said waste water treating device may comprises a plurality of said perforated containers.

Said perforated containers may be in contact with one another.

Said microorganism carriers may have different average grain sizes and be alternately stacked in said containers.

Said microorganism carriers may have different average grain sizes and be concentrically packed in said containers.

In said waste water treating apparatus, the ratio of diameter of said microorganism carriers having different average grain size may preferably be 1 : 1.5 - 2.5.

Said microorganism carriers may be wood chips.

Said containers may be mesh baskets.

The mesh size (air gap) of said mesh baskets may preferably be 3 mm - 7 mm.

Said containers may be unglazed containers.

Said split-flow device may include a cleaning device, and waste water from the cleaning device may be returned to the flow rate controlling tank or the precipitation-separation tank.

### BRIEF DESCRIPTION OF THE DRAWINGS

These and other objects of the invention will be explained in the description below, in connection with the accompanying drawings, in which:
FIG. 1 is a front perspective view showing the structure of an apparatus comprising an organic waste water treatment device in accordance with one embodiment of the present invention, for treating waste water containing a solid substance;
FIG. 2 is a partial perspective view of a solid-liquid separating device 400;
FIG. 3(a), (b) and (c) are diagrams showing the operation of the solid-liquid separating device 400; and
FIG. 4 (a) and (b) are schematic views showing the decomposing ability and air amount (gap amount) in each layer (each mesh basket container) in a secondary treating device 500 comprising plural levels of cylindrical mesh basket containers according to (a) the present invention and (b) a related art.

### DETAILED DESCRIPTION OF THE PREFERRED EMBODIMENT

A first embodiment of the present invention will be described with reference to the accompanying drawings.

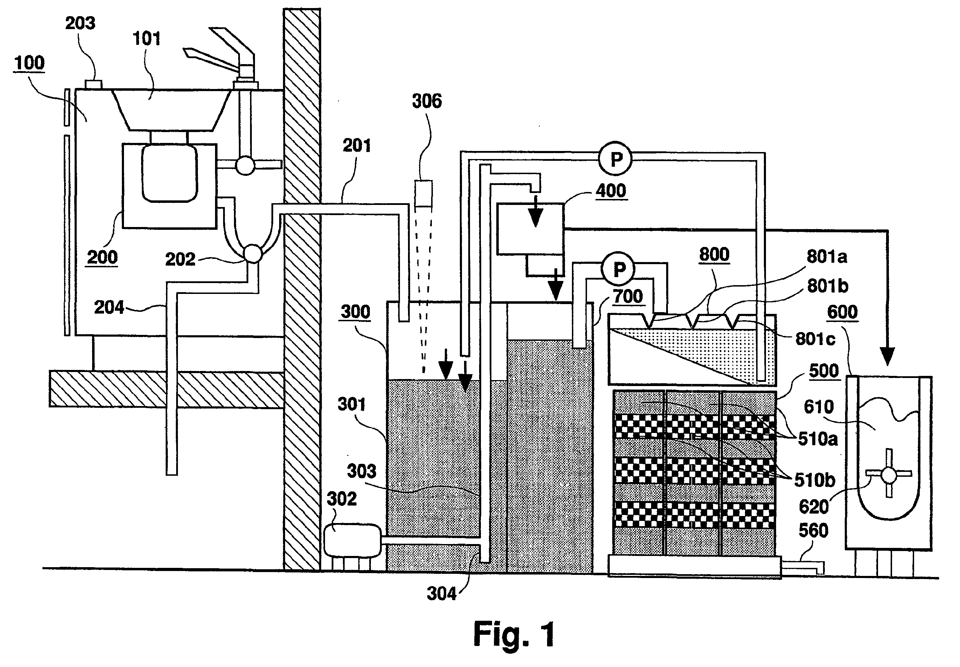

Fig. 1 shows the structure of a waste treatment system according to the present embodiment, the system being connected to a kitchen counter.

Referring to Fig. 1, the waste treatment system comprises a disposal 200 for finely grinding, mulching, or similarly reducing into a fine composition waste discharged from a sink 100 of the kitchen counter 100; a flow rate controlling tank 300 into which flows a mixture from the disposal of a solid substance such as ground garbage or the like and a liquid substance such as kitchen waste water or the like ; a solid-liquid separating device 400 for separating the mixture into a solid substance and a liquid substance; a precipitation-separation tank 700 for precipitating fine particles in the liquid component; a split-flow device 800 for distributing a supernatant (which will be described later) pumped from the precipitation-separation tank 700; a secondary treating device 500 for performing a purification treatment of the liquid substance; and a solid substance treating device (composting device) 600 for composting the solid substance.

The disposal 200, which includes an electromagnetic valve 202 and a start switch 203, is disposed under the sink 101 while the solid-liquid separating device 400, the precipitation tank 700, the split-flow device 800, the secondary treating device 500 and the solid substance treating device 600 are housed in a body case (not shown) and is located outside the house. The mixture discharged from the disposal 200 is temporarily introduced into the flow rate controlling tank 300 through an introduction pipe 201.

When only water, which is harmless to the environment when discharged into sewerage, is to be directly discharged through the discharge pipe 204, the start switch is turned OFF to inhibit start of the disposal 200. The electromagnetic valve 202 then causes the disposal 200 and the discharge pipe 204 to communicate each other, so that water from the sink 101 is discharged into sewerage.

On the other hand, when garbage or waste is to be discharged along with water from the sink 101, it is not desirable to discharge such water containing waste untreated, and therefore the start switch 203 is turned ON to start the disposal 200. The electromagnetic valve 202 then causes the disposal 200 and the introduction pipe 201 to communicate with each other, so that composting and purification, as will be described later, is performed.

The flow rate controlling tank 300 comprises a storage tank 301 for storing the mixture introduced through the introduction pipe 201, a solid substance transporting air lift pump 304 for transporting the mixture mainly composed of solid substances existing at the base portion of the storage tank 301 to the solid-liquid separating device 400 through the pipe 303, and a water level sensor 306 for detecting the water level of the mixture stored the storage tank 301. The air lift pump 304 comprises a blower 302 for blowing air for lifting and the air lift pipe 303 into the bottom of which air is supplied from the blower 302, so that the air lift pump 304 transfers the solid substances at the lower part of the air lift pipe 303 upward, together with the liquid, to the upper end portion, from which the liquid and solid are supplied to the solid-liquid separating device 400. At this point, when the water level of the storage tank 301 is high, the water pressure at the bottom of the storage tank 301 is high, so that a large amount of solid substances and liquid are supplied to the solid-liquid separating device 400 by a driving force from the air lift pump 304. On the other hand, when the water level of the storage tank 301 is low, the water pressure at the bottom of the storage tank 301 is reduced so that a small amount of the solid substances and liquid are supplied to the solid-liquid separating device 400 by driving the air lift pump 304. Therefore, the driving period of the air lift pump 304 or the amount of air supplied to the air lift pump 304 is controlled in order to supply a substantially constant amount of solid substances and liquid to the solid-liquid separating device 400 regardless of the water level of the storage tank 301.

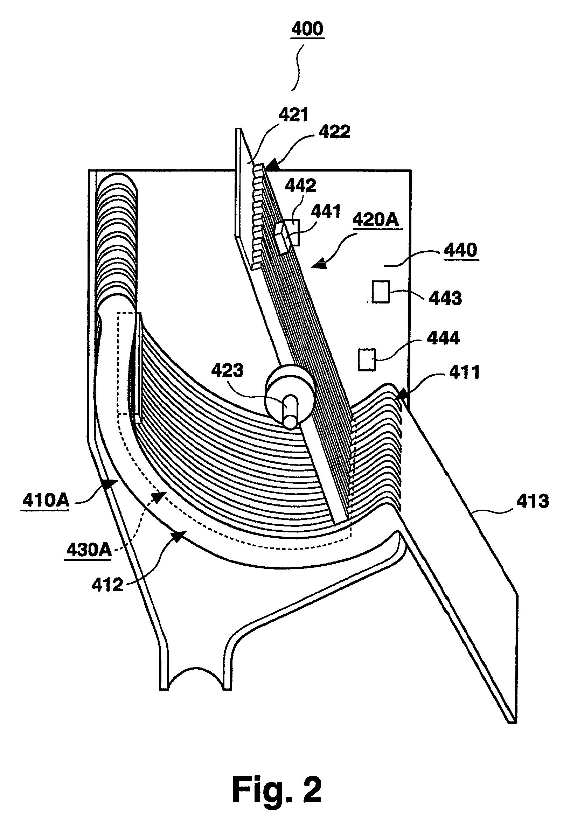

As shown in Fig. 2, the solid-liquid separating device 400 comprises a first slit portion 410A having a great number of drain slots 411 formed therein, a transport portion 420A for transporting the mixture having been subjected to solid-liquid separation, a second slit portion 430A which is connected to an end of the transport portion such that it can swing between the drain slots for accelerating solid-liquid separation of the introduced mixture, and a position detection portion 440 for detecting the position of the transport portion 420A. The first slit portion 410A, the transport portion 420A, and the second slit portion 430A are integrally resin molded by plastic or the like.

The first slit portion 410A has arc-shaped drain teeth 412 for screening and draining the mixture of solid and liquid introduced through the introduction pipe 201, and an introduction plate 413 for guiding the solid substances to the solid substance treating device 600 by causing the mixture having been subjected to draining (namely, solid substances in this case) to slide along the upper surface. Each of the drain slots 411 is formed between the drain teeth 412.

The transport portion 420A is fixed to a rotation shaft 423 coupled to a motor (not shown), and is composed of a transport plate 421 for transporting solid substances and ribs 422 provided on the rear surface of the transport plate 421. The ribs 422 are provided so as to provide the transport plate 421, which is formed of a thin plastic material, with sufficient strength to resist deformation.

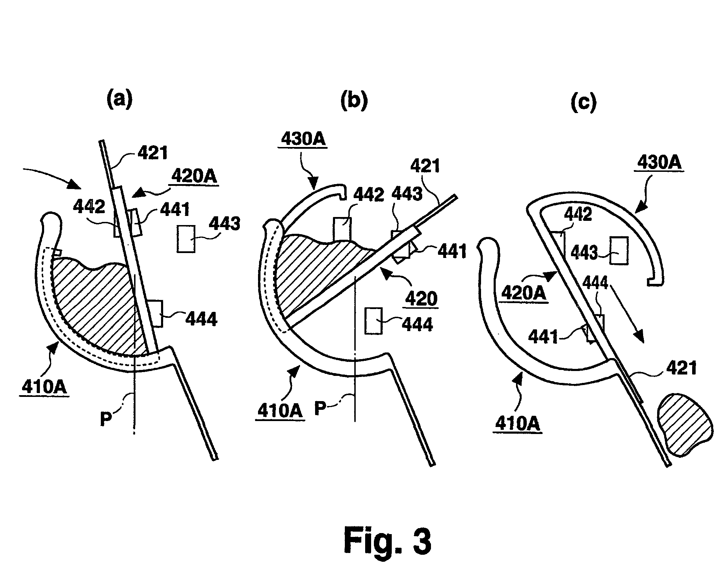

The mixture is transported through the air lift pump 304 to the portion of the transport portion 420A shown on the left side of the drawing, where liquid drips down through the drain slots 411 while the solid substances are accumulated on the drain teeth 412. The second slit portion 430A connected to end portion of the transport portion 420A is disposed within the drain slots 411, and swings within the drain slots 411 in accordance with the swinging movement of the transport portion 420A. In other words, by rotating the transport portion 420A back and forth within a predetermined angular range, the second slit portion 430A is also made to rotate, to thereby stir the solid substances on the drain teeth 412 for promoting the draining process. By rotating the transport portion 420A clockwise with appropriate frequency, the solid substances on the drain teeth 412 are temporarily pushed upward by the transport plate 421 and then drop down onto the introduction plate 413, which then transports the solid substances to the solid substance treating device 600. This operation state of the solid-liquid separating device 400 is shown in Fig. 3.

Referring back to Fig. 1, numeral 700 denotes the precipitation-separation tank 700 which precipitates fine particulate from the liquid component supplied from the solid-liquid separating device 400, and numeral 500 denotes the secondary treating device functioning as an organic waste water treating device for treating organic waste water (primary treated water) which is discharged from the solid-liquid separating device 400. A supernatant obtained by precipitation separation in the precipitation-separation tank 700 is supplied to the secondary treating device 500. On the other hand, precipitants accumulated at the bottom of the precipitation-separation tank 700 are appropriately returned to the flow rate controlling tank 300.

The split-flow device 800 distributes the supernatant supplied from the precipitation-separation tank 700 into the secondary treating device 500. In this example, the supplied liquid is evenly divided into split flows by means of an overflow weir having a plurality of triangular cut portions formed therein.

The secondary treating device 500 has at least two perforated cylindrical mesh basket containers 510a, 510b having a diameter of 15 cm, the mesh basket containers vertically arranged in contact with each other. In Fig. 1, four containers 510a and three containers 510b are alternatively stacked, the total heights of a container 510a and a container 510b being approximately 15 cm, and the stacks are arranged in three parallel rows.

The purpose for vertically arranging the cylindrical mesh basket containers 510a, 510b in upper and lower levels in contact with each other is as follows. When the garbage treatment apparatus is used for an extensive time, the container 510a at the upper level will eventually become clogged with solid substances, thereby preventing water from flowing through the container. To deal with this problem, maintenance of the containers is necessary. In this maintenance, the container 510a at the upper level is replaced by the container 510b at the lower level, and a new microorganism carrier is supplied at the lower level. The vertical two-level arrangement according to the present embodiment facilitates such maintenance. In the example of Fig. 1, when replacing the containers, a new container 510b is added at the lowermost level while the container 510a at the uppermost level is removed.

It should be noted that any number (2 or more) of levels of the cylindrical containers 510a, 510b may be provided for similarly achieving the effects of the present invention.

The ratio of the average grain size of the microorganism carriers within the cylindrical containers 510a and 510b is set to 1 : 1.5 ∼ 2.5, because when the ratio is greater than 1 : 1.5 - 2.5 there is a possibility that a microorganisms carrier having a smaller average grain size would enter a microorganism carrier having a larger average grain size.

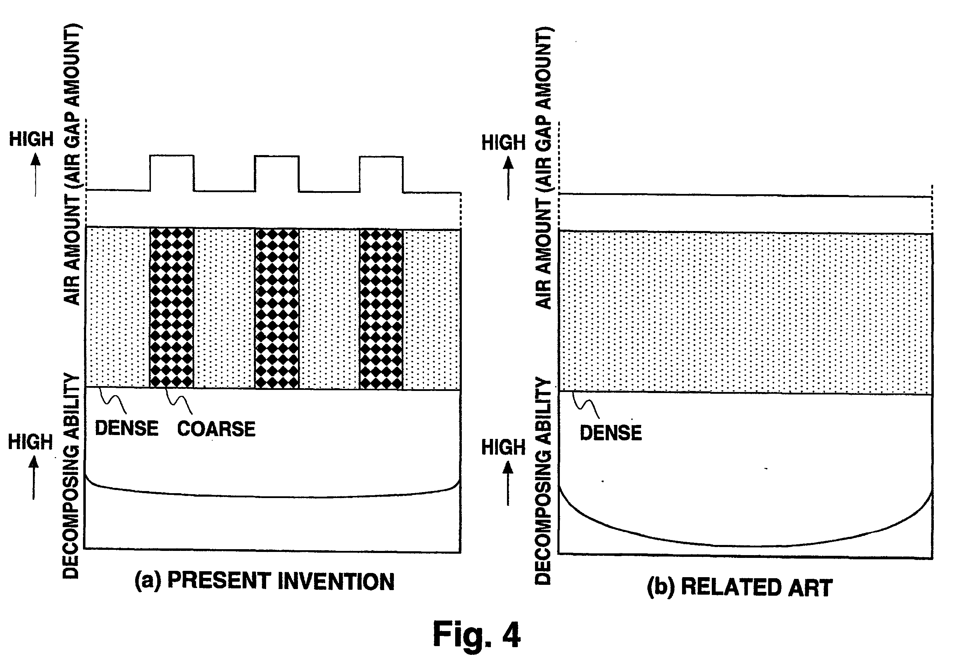

Referring to Fig. 4, comparison results are shown for the decomposing ability and an air amount (an air gap amount) for each layer (each container) between (a) the secondary treating device 500 comprising a plurality of cylindrical mesh basket containers according to the present invention and (b) a secondary treating device 500 comprising a plurality of cylindrical mesh basket containers according to a prior art (b).

As can be seen from Fig. 4, in the prior art example (b), the secondary treating device 500 which comprises only dense microorganism carriers contains a small amount of air, and therefore activation of the microorganisms decreases near the center part of the secondary treating device 500, which leads to exhibit a low ability for treating microorganisms.

To the contrary, in the present invention (a) comprising alternate arrangement of dense and coarse microorganism carriers, although the dense microorganism carrier part contains a smaller amount of air, the coarse microorganism carrier part contains a larger amount of air, and therefore a larger amount of air is contained even at the center part of the secondary treating device 500. As a result, activation of the microorganisms is accelerated to increase treating ability for the microorganisms.

All the containers are packed with wood chips (microorganism carriers) composed of shavings of Japanese cedar, so that the secondary treating device 500 subjects organic components within the introduced organic waste water to an oxidation decomposing treatment by means of aerobic microorganisms living in the carriers.

In the example described, the mesh size (a gap) of the cylindrical mesh basket containers 510a, 510b is 3 ~ 7 mm, preferably 5 mm, and the size of wood chip is 2 ~ 10 mm.

In another configuration, in the cylindrical mesh basket containers 510a and 510b, the bottom 5 cm are packed with wood chips of 5 - 10 mm size and next 10 cm are packed with 2 ~ 4 mm wood chips.

In still another configuration, a central cylindrical (diameter: 5 cm) area in the cylindrical mesh basket containers 510a and 510b is packed with wood chips of 2 ~ 4 mm size, and areas above and below are packed with 5 ~ 10 mm wood chips.

Other types of containers other than mesh basket containers may be used as the cylindrical containers 510a, 510b, as long as they include holes through which air can pass. Unglazed containers may be used, for example.

Weirs (cutout portions) are formed in the split-flow device 800. The action of these weirs (cutout portions) allows the primary treated water to be evenly distributed into a plurality of secondary treating devices 500 located below. Specifically, in the example of Fig. 1, three cutout portions are provided, and a liquid overflowing through respective cutouts is separately extracted so as to obtain evenly split flow.

The weir (a cutout portion) of the split-flow device 800 is formed by cutting out a triangular piece having each side of 5 cm. It is preferable to appropriately vary the side length of the triangular piece in proportion to an amount of liquid to be supplied from the precipitation-separation tank 700. If the weirs are small even when a large amount of the liquid component is supplied, the liquid would overflow from the split-flow device 800 or the weirs would be clogged with fine particles contained in the liquid supplied from the precipitation-separation tank 700.

Further, the portion of the split-flow device 800 before the weirs preferably has an inclined bottom so as to allow cleaning when sludge is accumulated thereon, and a pump is provided for pumping the accumulated sludge. The waste water after cleaning is returned back to the flow rate controlling tank 300 or the precipitation-separation tank 700.

A discharge pipe 560 is connected to the bottom of the containers 500 for discharging treated water.

In the secondary treating device 500 having the structure as described above, the primary treated water in the solid-liquid separation device 400 is diffused on the center part of the surface of the cylindrical mesh basket containers 510a, flows downward in contact with the microorganisms which decompose the organic components in the primary treated water, and is finally discharged through the discharge pipe 560 out of the garbage treating apparatus.

In particular, because the supplied liquid is split by the split-flow device 800, the primary treated liquid can be evenly supplied from above the secondary treating device 500. As a result, treatment can be improved by employing the entire secondary treating device 500. In the example shown in Fig. 1, stacks comprising the mesh basket containers 510a, 510b are provided in three rows, and, therefore, the split-flow device 800 splits the supplied primary treated water into three flows, which are then evenly supplied to the respective mesh basket containers 510a.

The solid substance treating device 600 comprises a treating tank 610 for storing the solid substances introduced after solid-liquid separation, a stirrer 620 for stirring the solid substances, and a heater or the like (not shown).

The treating tank 610 contains carriers composed of wood chips or shavings or the like and activated carbon for cultivating microorganisms. The microorganisms decompose the solid substances into carbon dioxide and water so as to produce compost.

The solid substances and the carriers are mixed by the stirrer 620 while air is introduced inside the treating tank 610 which is maintained at a predetermined temperature (30°C ∼ 40°C in the present embodiment) by the heater, to thereby accelerate activation of the microorganisms or the like.

The operation of the garbage treating apparatus having the above structure will next be described.

When waste is to be treated, the start switch 203 is turned ON to start the disposal 200. As a result, the electromagnetic valve 202 is operated so that garbage discharged from the sink 101 is ground in the disposal 200 and is then introduced into the storage tank 301 through the introduction pipe 201.

By configuring the introduction pipe 201 such that it is inclined by an appropriate amount, it is possible for the pulverized garbage to be transported into the storage tank 301 without employing a separate drive force or the like.

When waste treatment is not required (for example, when fresh water is discharged), the start switch 200 is left OFF. In this state, the electromagnetic valve 202 would not operate, so that waste water or the like flows directly into the discharge pipe 204.

Most of the solid substances contained in the mixture introduced into the storage tank 301 precipitate and collect at the bottom of the storage tank 301. The air lift pump 304 is then driven so that substantially a fixed amount of solid substances and liquid can be supplied to the solid-liquid separating apparatus 400, regardless of the water level of the storage tank 301 detected by the water level sensor 306. The mixture containing a large amount of solid substances stored at the bottom of the storage tank 301 is transported through the pipe 303 to the solid-liquid separation device 400 by means of the solid substance transporting air lift pump 304.

At this point, the state of the transport portion 420A of the solid-liquid separating device 400 is as shown in Fig. 2. Specifically, the standby switch 442 is operated by the magnet 441 so that the transport plate 421 awaits introduction of the mixture at a standby position.

Accordingly, the mixture transported from the flow rate controlling tank 300 contacts the transport plate 421 and loses energy and speed, such that it accumulates on the first slit portion 410A.

The transport portion 420A and the second slit portion 430A swing back and forth by means a motor (not shown) between the states shown in Fig. 3(a) and Fig. 3(b) by the action of the standby position switch 442 and the swing limit position switch 443. This swinging movement causes the agglomeration of the mixture containing the separated solid substances to be stirred for draining, so that solid-liquid separation can be performed with high efficiency.

Because the optimum number of swing depends on the type of solid substance, the number of swinging movements for each introduction of the mixture can preferably be set to a rate optimized for the introduced substance. However, from the standpoint of solid-liquid separation efficiency, a range of 5 ~ 40 times is preferable.

After a predetermined number of swinging movements, the transport portion 420A rotates until the solid substance discharge position switch 444 operates, so that the solid substances separated from the liquid component is placed into the solid substance treating device 600.

The treating tank 610 of the solid substance treating device 600 contains the carriers composed of wood chips or shavings and activated carbon for cultivating microorganisms. The solid substances having been subjected to solid-liquid separation and introduced into the treating tank are decomposed for composting, and then bagged or the like for discharge.

The lower the water content in the solid substances separated from the liquid component in the solid-liquid separation device 400, the shorter the time required for composting the solid substances. If it is desired that treatment be completed within a fixed amount, a the capacity of the treating tank 610 can be designed sufficiently large for treating the solid substances having a high water content.

According to the present embodiment, however, the solid-liquid separation efficiency of the solid-liquid separating device 400 is improved as described above, so that the treating tank 610 can be downsized and the cost for the whole apparatus can be reduced.

As described above, according to the present invention, it is possible to provide a solid-liquid separating device capable of solid-liquid separation in a short time while reducing the amount of solid substances passing through the drain slots 411, and a garbage treating system using the same.

As clearly described above, the present invention provides an advantage in that organic water such as a liquid output from a disposal or kitchen waste water can be effectively treated by means of preferable aerobic treatment using natural diffusion of oxygen and without supplying forced air circulation by an air pump as in the conventional garbage treating apparatus.

While the preferred embodiment of the present invention has been described using specific terms, such description is for illustrative purposes only, and it is to be understood that changes and variations may be made without departing from the spirit or scope of the appended claims.

## Claims

1. A waste treatment apparatus comprising:
a disposal for mechanically reducing waste discharged from a kitchen;
a flow rate controlling tank for temporarily storing a mixture of the waste reduced by said disposal and kitchen waste water;
a solid-liquid separating device for separating the mixture supplied from the flow rate controlling tank into a solid component and a liquid component;
a composting device for converting the solid component separated by the solid-liquid separating device into a compost;
a precipitation-separation tank for precipitating fine particles in the liquid component supplied from the solid-liquid separating device;
a split-flow device for distributing the liquid component supplied from the precipitation-separation tank; and
a waste water treating device for biologically treating the liquid component supplied from the split-flow device to thereby produce treated water.

2. A waste treatment apparatus according to claim 1, wherein
said waste water treating device comprises a tank containing microorganism carriers, and
said liquid component is introduced into the tank, subjected to a biological treatment in the tank, and then discharged out of said waste treatment apparatus.

3. A waste treatment apparatus according to claim 2, wherein said waste water treating device has perforated containers containing said microorganism carriers.

4. A waste treatment apparatus according to claim 3, wherein said waste water treating device comprises a plurality of said perforated containers.

5. A waste treatment apparatus according to claim 3 or 4, wherein said perforated containers are in contact with one another.

6. A waste treatment apparatus according to any of claims 3 to 5, wherein said microorganism carriers comprise grains of a plurality of average grain sizes which are alternately stacked in said containers.

7. A waste treatment apparatus according to any of claims 3 to 5, wherein said microorganism carriers comprise grains of a plurality of average grain sizes which are concentrically packed in said containers.

8. A waste treatment apparatus according to any of claims 6 to 7, wherein, in said waste water treating apparatus, the ratio of diameters of said microorganism carriers having different average grain size is 1 : 1.5 - 2.5.

9. A waste treatment apparatus according to any of claims 2 to 8, wherein said microorganism carriers are wood chips.

10. A waste treatment apparatus according to any of claims 3 to 9, wherein said containers are mesh baskets.

11. A waste treatment apparatus according to claim 10, wherein the mesh size (air gap) of said mesh baskets is 3 mm ∼ 7 mm.

12. A waste treatment apparatus according to any of claims 3 to 8, wherein said containers are unglazed containers.

13. A waste treatment apparatus according to any of claims 1 to 12, wherein said split-flow device includes a cleaning device, and waste water from the cleaning device is returned to the flow rate controlling tank or the precipitation-separation tank.
